# EUROPÄISCHE PATENTSCHRIFT

(11) **EP 2 135 072 B1**
(45) Veröffentlichungstag und Bekanntmachung des Hinweises auf die Patenterteilung: **17.02.2016**
(21) Anmeldenummer: 08716527.0
(22) Anmeldetag: 13.03.2008
(51) Int. Cl.: G01N 33/18, C12Q 1/02

(54) **VORRICHTUNG UND VERFAHREN ZUR ONLINE-KONTROLLE VON TRINKWASSER AUF HUMANVERTRÄGLICHKEIT INNERHALB EINES TRINKWASSERVERSORGUNGSNETZES**
DEVICE AND METHOD FOR THE ONLINE CONTROL OF DRINKING WATER ON HUMAN TOLERANCE WITHIN A DRINKING WATER SUPPLY NETWORK
DISPOSITIF ET PROCÉDÉ POUR LE CONTRÔLE EN LIGNE D'EAU POTABLE POUR L'APTITUDE À LA CONSOMMATION HUMAINE À L'INTÉRIEUR D'UN RÉSEAU DE DISTRIBUTION D'EAU POTABLE

(30) Priorität: 14.03.2007 DE 102007012970
(43) Veröffentlichungstag der Anmeldung: 23.12.2009
(73) Patentinhaber: Fraunhofer-Gesellschaft zur Förderung der angewandten Forschung e.V., 80686 München (DE)
(72) Erfinder: BEYERER, Jürgen, 69234 Dielheim (DE)
(74) Vertreter: Rösler, Uwe
(86) Internationale Anmeldenummer: PCT/EP2008/002030
(87) Internationale Veröffentlichungsnummer: WO 2008/110375

(56) Entgegenhaltungen:
- WO-A-01/53517
- WO-A-96/14563
- DE-A1- 4 332 163
- DE-A1- 10 226 731
- DE-U1- 29 513 115

## Beschreibung

### Technisches Gebiet

Die Erfindung bezieht sich auf eine Vorrichtung sowie ein Verfahren zur Online-Kontrolle von Trinkwasser auf Humanverträglichkeit innerhalb eines Trinkwasserversorgungsnetzes mit wenigstens einer Trinkwasserspeisestelle, mit der eine Vielzahl von Trinkwasser führenden Leitungen mittel- oder unmittelbar verbunden sind, die zu dezentralen Trinkwasserverbraucherstellen führen.

### Stand der Technik

Infrastruktursysteme gehören zu den häufig angegriffenen Zielen des internationalen Terrorismus. Beispiele sind Verkehrsinfrastruktur, Elektroenergieversorgungssysteme oder Öl- und Gasleitungen, die bisher in der Regel mit Sprengstoff angegriffen wurden. Eine Sonderstellung unter den Infrastruktursystemen nimmt in diesem Kontext die Trinkwasserversorgung ein. Neben Sprengstoff als Angriffsart kommen hierbei auch Angriffe mit radioaktiven Substanzen, mit Krankheitserregern und mit Giftstoffen (ABC-Angriffe) in Frage. ABC-Angriffe lassen sich geräuschlos ausführen und werden in der Regel erst sehr spät an ihren Folgen (lokal gehäuft auftretende Todesfälle, Epidemien, gehäuft auftretende Krankheitsbilder etc.) erkannt. In der Regel ist dabei weder das Angriffsmaterial noch der Angriffsort (Ort, an dem das Angriffsmaterial in das Trinkwasser eingeschleust wurde) bekannt. Der Nachweis tödlicher Krankheitserreger oder hochtoxischer Substanzen im Trinkwasser ist auf Speziallabors und entsprechende Ausrüstung und Expertise beschränkt und kann unter Umständen Tage oder Wochen dauern, wenn denn überhaupt im Trinkwasser gesucht wird. Erschwerend kommt hinzu, dass zu den bewirkten Sach- und Gesundheitsschäden ein Angriff auf die Trinkwasserversorgung eine verheerende psychologische Wirkung hat. Bei ABC-Angriffen auf die Trinkwasserversorgung kommt daher einer schnellen, möglichst on-line fähigen Detektion eines erfolgten Angriffs eine Schlüsselfunktion bei der Schadensbegrenzung und Schadensbewältigung zu.

Während der Nachweis radioaktiver Strahlung über deren ionisierende Wirkung (Geigerzähler etc.) zumindest prinzipiell schnell und zuverlässig möglich ist, gestaltet sich die Suche nach bestimmten Krankheitserregern oder nach einem der zahlreichen möglichen Giftstoffe (Biotoxine, Kampfstoffe, synthetische Giftstoffe etc.) zur sprichwörtlichen Suche nach der Stecknadel im Heuhaufen, selbst dann wenn von Anfang an das Trinkwasser in die Ursachensuche einbezogen wird.

Eine schnelle Detektion eines erfolgten Angriffs mit B- oder C-Substanzen sollte zumindest die Bestätigung des Angriffs innerhalb einer kurzen Zeitspanne (< 2 Stunden) erbringen, nur in diesem Zeitrahmen ist es noch möglich, wenigstens Teile der betroffenen Bevölkerung rechtzeitig zu warnen oder die Wasserversorgung abzustellen und Proben für anschließende Laboruntersuchungen zu gewinnen. Bei längeren Detektionszeiten muss davon ausgegangen werden, dass nicht nur die Schadwirkung kaum mehr aufgehalten werden kann, sondern auch, dass der Nachweis durch Verdünnung und Verbrauch immer schwieriger wird. Im ungünstigsten Fall findet man die Ursache der Todesfälle oder gesundheitlichen Beschwerden im Verteilernetz gar nicht mehr, da sie durch den Wasserverbrauch bereits ausgespült werden.

Bei Detektionszeiten in der genannten Größenordnung von mehr als 2 Stunden kommt eine stoffspezifische Analytik oder eine Speziesidentifizierung nicht in Frage. Man muss sich zusammen auf die Wirkung des ins Trinkwasser eingeschleusten Materials auf den menschlichen Organismus beschränken, bei Giften also auf die direkte Schädigung des Organismus durch Vergiftung.

Die DE 10 2004 048 316 A1 beschreibt ein Quasi-Online-Verfahren und eine Anordnung zur qualitativen Bestimmung des Verkeimungsgrades in offenen Kühl- und Klimaanlagen. Dabei soll ermittelt werden, ob ein empfohlener Grenzwert von 10⁵ KbE (Kolonie bildende Einheiten) überschritten wird, da dann die hohe bakteriologisch Belastung zu Biofilmen, Korrosionen, Wärmeübertragungsverlusten in den Kühl- und Klimaanlagen bzw. zu Gesundheitsproblemen führen kann. Dazu wird belastetes Umlaufwasser bzw. als Referenz Trinkwasser einer Mikrodurchflussmesskammer ventilgesteuert zugeführt. Unter luftdichtem Abschluss wird die dynamische Sauerstoffzehrung über eine Sauerstoffsonde ermittelt. Unter der Voraussetzung, dass überwiegend heterotrophe Mikroorganismen sowie überwiegend gut biologisch abbaubare Substanzen das Umlaufwasser kontaminieren, wird ein qualitativer Zusammenhang zwischen Gesamtkeimzahl und biologischem Sauerstoffbedarf angenommen.

Die DE 295 13 115 U1 offenbart einen Biosensor zur Bestimmung der im Ab- oder Brauchwasser von Kläranlagen enthaltenen biologischen Substanzen, die von Mikroorganismen, z.B. bei der biologischen Abwasserreinigung, abgebaut werden. Der Biosensor erfasst die Sauerstoffzehrung, d.h. den Sauerstoffbedarf, den Mikroorganismen beim Abbau der im Abwasser enthaltenen biologischen Substanzen aufweisen. Der ermittelte biologische Sauerstoffbedarf - ein Summenparameter, der ein Maß für die Verschmutzung von Abwässern ist, dient als Messwert für steuernde Eingriffe oder Regelungen von biologischen Abwasserreinigungsprozessen.

Die DE 102 26 731 A1 zeigt eine Vorrichtung und Verfahren zur Online-Detektion biologischer Kontaminanten und deren Reste in Trinkwasser. Dazu werden Trinkwasserproben durch zwei parallel geschaltete Messzellen gepumpt. Die Messzellen enthalten jeweils eine oder mehrere Kollektorplatten aus lichtdurchlässigem Material, auf deren Oberfläche die jeweils zu beobachtenden Keime selektiv gebunden werden. Die Bindung erfolgt über Vermittlung spezieller Proteinkomplexe, insbesondere über monoklonale Antikörper. An den Kollektorplatten haftende Keime oder deren Reste können durch spektrophotometrische oder fluoreszenzphotometrische Verfahren erfasst werden. Auf dieser Weise kann Trinkwasser selektiv auf das Vorhandensein und die Konzentration von bestimmten Keimen untersucht werden. Schädliche Keime, für die keine Kollektorplatte mit entsprechenden monoklonalen Antikörpern vorgesehen ist, werden nicht erfasst. Toxische Stoffe nicht biologischer Art können nicht detektiert werden.

Die DE 43 32 163 A1 zeigt ein Verfahren und ein Gerät zur Schadstoffanalyse von Gewässerproben. Dazu wird eine zu analysierende Gewässerprobe mit einer Algensuspension gemischt. Nach einer vorgegebenen Reaktionszeit wird das Gemisch mit einem schwachen und modulierten Messlicht sowie mit einem aktiven, stärkeren, unmodulierten Dauerlicht bestrahlt. Die Schadstoffanalyse basiert auf Messwerten des vom Gemisch emittierten Fluoreszenzlichts sowie der Sauerstoffentwicklung im Gemisch. Diese Messwerte werden mit Messwerten verglichen, die für in bekannter Weise belastete Referenzwässer ermittelt wurden.

Die WO 01/53517 A1 zeigt eine Vorrichtung zur automatischen Messung der Toxizität von Gewässerproben. Die Gewässerproben werden mit lumineszenten Mikroorganismen versetzt. Zur Beurteilung der Toxizität wird das emittierte Fluoreszenzlicht der Mikroorganismen erfasst.

### Darstellung der Erfindung

Es liegt die Aufgabe zugrunde eine Vorrichtung, die an wenigstens einem vom Trinkwasser durchströmbaren Trinkwasserleitungsabschnitt innerhalb eines Trinkwasserversorgungsnetztes vorsehbar ist, sowie ein Verfahren zur Online-Kontrolle von Trinkwasser auf Humanverträglichkeit innerhalb eines Trinkwasserversorgungsnetzes mit wenigstens einer Trinkwasserspeisestelle, mit der eine Vielzahl von Trinkwasser führenden Leitungen mittel- oder unmittelbar verbunden ist, die zu dezentralen Trinkwasserverbraucherstellen führen, derart weiterzubilden, dass eine schnelle, sichere und zuverlässige Überprüfung des Trinkwasser möglich wird. Die hierfür zu treffenden Maßnahmen sollen Ressourcen schonend, automatisch arbeitend und möglichst kostenminimiert realisierbar sein.

Die Lösung der der Erfindung zugrunde liegenden Aufgabe ist im Anspruch 1 und 2, sowie im Anspruch 8 und 9 angegeben.

Den Erfindungsgedanken vorteilhaft weiterbildende Merkmale sind den Unteransprüchen sowie der weiteren Beschreibung unter Bezugnahme auf die Ausführungsbeispiele zu entnehmen.

Lösungsgemäß zeichnet sich eine Vorrichtung nach den Merkmalen des Oberbegriffes 1 dadurch aus, dass ein semipermeables Begrenzungsmittel vorgesehen ist, das derart in den Trinkwasserleitungsabschnitt einbringbar ist, dass ein zumindest in Strömungsrichtung einseitig begrenztes Durchströmungsvolumen erzeugbar ist, in das wenigstens ein biologische Mikroorganismen enthaltendes Indikatormedium eingebracht ist, das von dem Begrenzungsmittel innerhalb des Durchströmungsvolumen lokalisierbar ist, d.h. durch das Begrenzungsmittel innerhalb des Durchströmungsvolumens gegen die Strömungsrichtung zurückgehalten wird. Ferner ist zur optischen Erfassung des Indikatormediums außerhalb des Durchströmungsmediums eine erste Sensoreinheit vorgesehen, die als bildgebende Sensoreinheit ausgebildet ist und die in Kommunikation mit einer ein Bildauswertemodul aufweisenden, Auswerteinheit steht, in der Seitens der Sensoreinheit generierte Sensorsignale unter Zugrundelegung wenigstens einer Auswertevorschrift auswertbar sind und mittels der bei Eintreten eines vorgebbaren Entscheidungskriteriums ein Signal erzeugbar ist.

Alternativ zur Überwachung der Trinkwasserqualität, bei der das Indikatormedium zumindest in einen Teilstrom des Trinkwassers eingebracht wird, wie vorstehend vorgeschlagen, ist es gleichfalls möglich die Überwachung durch kontinuierliche Trinkwasserprobenentnahme vorzunehmen. Für diesen Fall zeichnet sich eine alternative lösungsgemäße Vorrichtung dadurch aus, dass wenigstens eine automatisch verschließbare Ableitung vorgesehen ist, mittels der portionsweise sowie unter Vorgabe eines Zeittaktes eine Trinkwasserprobe aus dem Trinkwasserversorgungsnetz ausspeisbar ist. Es ist wenigstens ein bevorratbares Gefäß vorgesehen, das mit einer Manipulatoreinheit, bspw. mittels Förder- oder Robotertechnik bereitgestellt wird und in das die Trinkwasserprobe über die Ableitung eingebracht wird. Über eine Zugabeeinheit ist im Weiteren ein biologische Mikroorganismen enthaltendes Indikatormedium in das mit der Trinkwasserprobe gefüllte Gefäß einbringbar. Zur optischen Erfassung des Indikatormediums ist außerhalb des Gefäßes eine erste Sensoreinheit vorgesehen, die als bildgebende Sensoreinheit ausgebildet ist. Eine mit der Sensoreinheit in Kommunikation stehende, ein Bildauswertemodul aufweisende Auswerteinheit vermag letztlich die

Seitens der Sensoreinheit generierten Sensorsignale unter Zugrundelegung wenigstens einer Auswertevorschrift auszuwerten und bei Eintreten eines vorgebbaren Entscheidungskriteriums ein Signal zu erzeugen, das bspw. als Alarmsignal dient, sofern die Trinkwasserqualität unter eine definierte Toleranzgrenze fällt.

In beiden der vorstehend bezeichneten Vorrichtungsalternativen gilt es als Indikatormedium ein auf mögliche B (biologisch)- oder C (chemisch)-Angriffsmaterialien sensibles Medium einzusetzen, das eine vergleichbare oder vorzugsweise höhere Empfindlichkeit gegenüber einem potentiellen Angriffsmaterial besitzt, als der menschliche Organismus, der gleichsam auch bei einem derartigen Angriff der toxikologischen Wirkung des vergifteten bzw. kontaminierten Leitungswasser im Wege des normalen Verbrauches ausgesetzt würde.

Somit kommt als Indikatormedium biologische Organismen oder Lebensgemeinschaft von Organismen in Frage, deren biologische bzw. toxikologische Wirkungen denen des menschlichen Organismuses möglichst nahe kommen, wobei die Reaktionszeit auf B- oder C-Angriffsmaterialien auch kürzer sein können, als beim menschlichen Organismus. Die im Weiteren als Indikatororganismen bezeichneten, einem biologischen Stoffwechsel unterliegenden Organismen weisen vorzugsweise eine makroskopische Größe auf, durch die die Organismen vorzugsweise in vereinzelter Form aber insbesondere durch Zusammenlagerung einer Vielzahl derartiger Organismen von einer bildgebenden Sensorik visuell erfassbar sind.

Die Indikatororganismen werden entweder innerhalb eines vorgegebenen Strömungsbereiches längs der Trinkwasserströmung oder innerhalb einer entnommenen Trinkwasserprobe in einem Gefäß von einer bildgebenden Sensoreinheit erfaßt, durch die die Indikatororganismen im Rahmen einer Bildauswertung hinsichtlich ihrer Farbe, Beweglichkeit und/oder räumlicher Verteilung untersucht werden. Mit Hilfe einer an die bildgebende Sensoreinheit verbundene Auswerteeinheit, in der die Sensorsignale unter vorgebbaren Bildauswerteregeln analysiert werden, kann aus den Bildern bzw. Sensorsignalen, bspw. anhand sich einstellender Farbänderungen, einer Veränderung der Vitalität und Beweglichkeit der einzelnen Organismen oder zu Kolonien zusammengefundenen Organismen, des Grades von möglicherweise absterbenden Mikroorganismen, einer sich einstellender Trübung und/oder eines Zerfalls von lokalen Ansammlungen ein Schädigungsmaß festgestellt werden, sowie eine sich einstellende Schädigungsgeschwindigkeit, über die man eine Skalierung der Sicherheit der Detektion des in Frage kommenden Angriffs ermitteln kann.

In einer bevorzugten Ausführungsform wird zur Verminderung der Falschalarmwahrscheinlichkeit parallel zur Untersuchung der mit dem Indikatororganen versetzten Trinkwasserprobe eine Probe mit unkontaminierten Wasser, d.h. 100%ig sauberem Wasser, mit Indikatororganismen versetzt und simultan beobachtet. Die mittels der beiden bildgebenden Sensoreinheiten gewonnenen Bildsignale werden der Bildauswerteeinheit zugeführt und im Rahmen einer Bildsignaldifferenzanlayse unterzogen. Durch den Vergleich beider jeweils durch die bildgebenden Sensoreinheiten beobachteten Indikatororganismen können natürliche Alterungserscheinungen, die nicht auf mögliche Fremdeinwirkungen zurückzuführen sind, ausgeschlossen werden,

Da mit dem Nachweis eines möglicherweise erfolgten B- oder C-Angriffs auf das Trinkwasser weitreichende, kostenträchtige und öffentlich wahrnehmbare Aktionen ausgelöst werden müssen, ist die Frage der Sicherheit des Nachweises und der Tragweite des Angriffes von besonderer Bedeutung. Die Meßwerte der lösungsgemäß vorgeschlagenen Vorrichtung, die als Toxizitätssensor aufgefaßt werden kann, müssen daher im Kontext mit Informationen des Versorgungsnetzes, d.h. Größe und Umfang des möglicherweise angegriffenen Trinkwasserversorgungsnetzes, gegenwärtiger Wasserverbrauch, Wassertransportzeiten bis zu den jeweiligen Trinkwasserverbraucherstellen, bis hin zu Daten des Einwohnermeldeamtes und des Gesundheitsamtes, gesehen werden. Aus der Gesamtzusammenschau der verfügbaren Informationen kann letztlich eine Situationsbewertung mit der Absicherung der Notwendigkeit von Maßnahmen abgeleitet werden.

Den vorstehend beschriebenen Vorrichtungen zur Online-Kontrolle von Trinkwasser auf Humanverträglichkeit innerhalb eines Trinkwasserversorgungsnetzes mit wenigstens einer Trinkwasserspeisestelle, mit der eine Vielzahl von Trinkwasser-führenden Leitungen mittel- oder unmittelbar verbunden sind, die zu dezentralen Trinkwasserverbraucherstellen führen, liegt ein gemeinsames lösungsgemäßes Verfahrensprinzip zugrunde, das sich dadurch auszeichnet, dass dem Trinkwasser innerhalb eines räumlich begrenzten Leitungsbereiches oder der Trinkwasserleitung periodisch portioniert entnommenen Trinkwasserproben biologische Mikroorganismen zugegeben wird, deren Vitalität mittels einer ersten bildgebenden Sensorik erfaßt wird, deren Bildsensorsignale im Rahmen einer Bildauswertung unter Zugrundelegung einer Auswertevorschrift analysiert werden und dass bei Entsprechen der Bildsensorsignale eines vorgebbaren Entscheidungskriteriums ein Signal erzeugt wird.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: schematisierte Darstellung zur Online-Kontrolle des in einer Leitung fließenden Trinkwassers, sowie
- Fig. 2: schematisierte Darstellung zur Online-Kontrolle periodisch portionierter Leitungswasserentnahme.

### Wege zur Ausführung der Erfindung, gewerbliche Verwendbarkeit

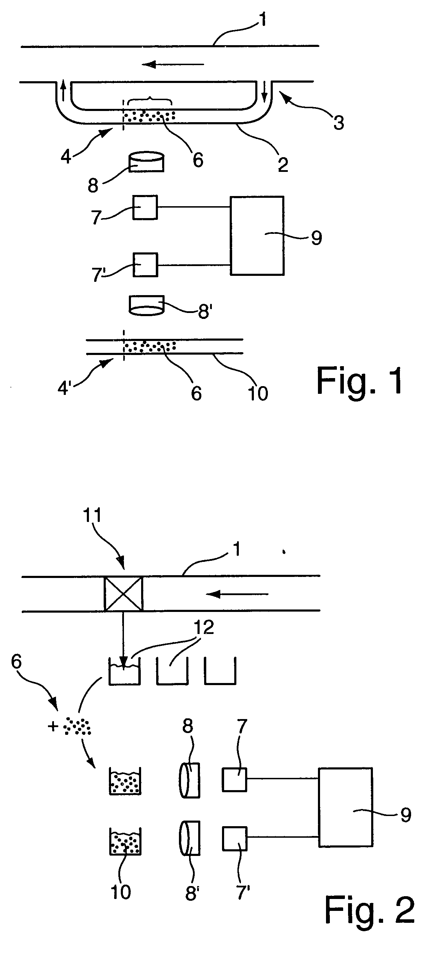

In Figur 1 ist eine schematisierte Darstellung einer Trinkwasser-führenden Leitung 1 dargestellt, längs der eine Bypass-Leitung 2 vorgesehen ist, in die über eine Verzweigungsstelle 3 ein Teil des in der Leitung 1 geführten Trinkwassers abgezweigt wird. Längs der Bypass-Leitung 2, deren Leitungsquerschnitt sehr viel kleiner dimensioniert ist als jener der Leitung 1, ist ein Separierungsmittel 4 vorgesehen, beispielsweise in Art eines Siebes, Gitters oder einer semipermeablen Membran, durch die das in die Bypass-Leitung 2 abgezweigte Leitungswasser vorzugsweise ungehindert hindurchtreten kann. In Strömungsrichtung vor dem Separierungsmittel 4 ist in einem Strömungsbereich 5 ein Indikatormedium 6 eingebracht, das aus biologischem, lebenden Organismen besteht, deren biologische Eigenschaften in Bezug auf die Wirkung von toxikologischen Kontaminationen vergleichbar jenen des menschlichen Organismuses sind.

Der mit den mikrobiologischen Organismen 6 versetzte Strömungsbereich 5 längs der Bypass-Leitung 2 wird mittels einer bildgebenden Sensoreinheit 7 detektiert. Da die mikrobiologischen Organismen 6 zum Teil für das bloße Auge nicht sichtbar sind, wird der Strömungsbereich 5 oder zumindest Teilbereiche des mit den Mikroorganismen 6 versetzten Strömungsbereichs 5 mit Hilfe einer Vergrößerungsoptik 8 auf die bildgebende Sensorik 7 abgebildet. Die seitens der bildgebenden Sensorik 7 abgegebenen Sensorsignale werden einer Auswerteeinheit 9 zugeführt, die die Bildsignale unter Maßgabe entsprechender Bildauswerteregeln auswertet. Die Online-fähige bildgebende Sensorik 7 vermag in Verbindung mit der Auswerteeinheit 9 rechnergestützt eine Bildauswertung durchzuführen und insbesondere Farbänderungen, Veränderung von Vitalität und Beweglichkeit bei den Mikroorganismen 6 festzustellen bzw. auszuwerten. Auch wird die visuell wahrnehmbar Trübung des Leitungswassers durch mögliches Ausscheiden der Mikroorganismen erfaßt sowie das Absterben oder Zerfall von Koloniebildungen detektiert.

Aus den von der bildgebenden Sensorik 7 aufgenommenen Bildern bzw. Sensorsignalen kann ein Schädigungsmaß und eine Schädigungsgeschwindigkeit abgebildet werden, über die man eine Skalierung der Sicherheit der Detektion des in Frage kommenden Angriffes ermitteln kann. Zur Minderung möglicher Falschalarme wird eine vor äußeren Eingriffen sichere Trinkwasserleitung 10 in gleicher Weise überwacht. Längs der Trinkwasserleitung 10, durch die 100%ig sauberes Trinkwasser, sogenanntes Referenztrinkwasser, strömt ist gleichsam ein Separierungsmittel 4' vorgesehen, gegenüber dem stromauf in gleicher Weise biologische Mikroorganismen 6 eingebracht sind. Mit Hilfe einer entsprechenden Abbildungsoptik 8' und einer bildgebenden Sensorik 7' werden die vor äußeren Einflüssen gesicherten Mikroorganismen 6 visuell überwacht. Die von den visuellen Sensoreinheiten 7 und 7' generierten Bildsignale werden innerhalb der Auswerteeinheit 9 im Wege einer Differenzbildanalyse untersucht und bei auftretenden Abweichungen ein entsprechendes Signal zur weiteren Alarmierung generiert. Mögliche natürliche Veränderungen an den Organismen, die nicht auf Fremdeinflüsse zurückzuführen sind, können auf diese Weise eliminiert werden.

Im Gegensatz zur kontinuierlichen Betriebsweise für eine Online-Kontrolle von Trinkwasser auf Humanverträglichkeit innerhalb eines Trinkwasserversorgungsnetzes gemäß dem in Figur 1 dargestellten Ausführungsbeispiel, sieht das in Figur 2 dargestellte Ausführungsbeispiel eine periodische Probenentnahme von Trinkwasser aus einer Trinkwasserleitung 1 an einer Entnahmestelle 11 vor. Die Trinkwasserprobenentnahme kann automatisch, beispielsweise mit einer Manipulatoreinheit, bspw. Robotereinheit vorgenommen werden, hierzu sind geeignete Gefäße 12 entsprechend zu bevorraten. Nach entsprechender Trinkwasserentnahme werden zur weiteren Untersuchung biologische Mikroorganismen 6 in das Gefäß 12 beigegeben, deren zeitlicher Verbleib innerhalb der Probe 12, in der das Trinkwasser strömungsfrei enthalten ist, mittels einer bildgebenden Sensorik 7 visuell erfaßt und überwacht wird. Auch in diesem Fall werden die biologischen Mikroorganismen 6 über eine Abbildungsoptik 8 und einer bildgebenden Sensorik 7 erfaßt und die hierdurch generierten Bildsignale einer Auswerteeinheit 9 übergeben. Gleichfalls wird zur Durchführung der bereits vorstehend beschriebenen Differenzbildmessung auch der zeitliche Verbleib von Mikroorganismen 6 innerhalb einer Referenztrinkwasserprobe 10' mit Hilfe einer entsprechenden Abbildungseinheit 8' und einer visuellen Sensorik 7' erfaßt. Diese Bildsignale werden gleichsam der Auswerteeinheit 9 zugeführt, die einer Differenzbildauswertung unterzogen werden. Der in Figur 2 dargestellte Batch-Betrieb kann gleichfalls nahezu kontinuierlich durchgeführt werden, es gilt lediglich eine geeignete periodische Zeittaktung zu wählen, mit der die zu untersuchenden Trinkwasserproben aus der Trinkwasserversorgungsleitung 1 entnommen werden.

### Bezugszeichenliste

- 1: Trinkwasserleitung
- 2: Bypass-Leitung
- 3: Abzweigestelle
- 4, 4': Separierungsmittel
- 5, 5': Strömungsbereich, Volumenbereich
- 6: biologische Mikroorganismen
- 7,7': visuelle Bildaufnahmesensorik
- 8,8': Abbildungsoptik
- 9: Auswerteeinheit
- 10: Referenztrinkwasserleitung
- 11: verschließbare Ableitung
- 12: Gefäß

## Patentansprüche

1. Vorrichtung zur Online-Kontrolle von Trinkwasser auf Humanverträglichkeit, die an wenigstens einem vom Trinkwasser durchströmbaren Trinkwasserleitungsabschnitt innerhalb eines Trinkwasserversorgungsnetzes vorsehbar ist, das wenigstens eine Trinkwasserspeisestelle aufweist, mit der eine Vielzahl von Trinkwasserleitungen (1) mittel- oder unmittelbar verbunden ist, die zu dezentralen Trinkwasserverbraucherstellen führen,
**dadurch gekennzeichnet, dass** die Vorrichtung ein biologische Mikroorganismen enthaltendes Indikatormedium umfasst, dass die Vorrichtung ein zum Einsatz in dem Trinkwasserleitungsabschnitt geeignetes, semipermeables Begrenzungsmittel (4) umfasst,
wobei im Einsatz das semipermeable Begrenzungsmittel(4) ein Durchströmungsvolumen des Trinkwasserleitungsabschnittes zumindest in Strömungsrichtung einseitig begrenzt, und wobei das Begrenzungsmittel (4) wenigstens das in das Durchströmungsvolumen einbringbare, biologische Mikroorganismen (6) enthaltende Indikatormedium lokalisiert, indem das Begrenzungsmittel (4) das Indikatormedium innerhalb des Durchströmungsvolumens gegen die Strömungsrichtung zurückhält,
dass zur optischen Erfassung des Indikatormediums außerhalb des Durchströmungsmediums eine erste Sensoreinheit (7) vorgesehen ist, die als bildgebende Sensoreinheit ausgebildet ist und die eine Vitalität der biologischen Mikroorganismen (6) erfasst, und
dass eine mit der Sensoreinheit (7) in Kommunikation stehende, ein Bildauswertemodul aufweisende, Auswerteinheit (9) vorgesehen ist, in der seitens der Sensoreinheit (7) generierte Sensorsignale unter Zugrundelegung wenigstens einer Auswertevorschrift auswertbar sind und mittels der bei Eintreten eines vorgebbaren Entscheidungskriteriums ein Signal erzeugbar ist.

2. Vorrichtung zur Online-Kontrolle von Trinkwasser auf Humanverträglichkeit, die an wenigstens einem vom Trinkwasser durchströmbaren Trinkwasserleitungsabschnitt innerhalb eines Trinkwasserversorgungsnetzes vorsehbar ist, das wenigstens eine Trinkwasserspeisestelle aufweist, mit der eine Vielzahl von Trinkwasserleitungen (1) mittel- oder unmittelbar verbunden ist, die zu dezentralen Trinkwasserverbraucherstellen führen,
**dadurch gekennzeichnet, dass** wenigstens eine automatisch verschließbare Ableitung (11) vorgesehen ist, mittels der eine Trinkwasserprobe aus dem Trinkwasserversorgungsnetz portionsweise ausspeisbar ist,
dass wenigstens ein bevorratbares Gefäß (12) vorgesehen ist, in das die Trinkwasserprobe über die Ableitung (11) einbringbar ist,
dass eine ein biologische Mikroorganismen (6) enthaltendes Indikatormedium enthaltende Zugabeeinheit vorgesehen ist, mittels der das die biologischen Mikroorganismen (6) enthaltende Indikatormedium in das mit der Trinkwasserprobe gefüllte Gefäß (12) einbringbar ist,
dass zur optischen Erfassung des Indikatormediums außerhalb des Gefäßes (12) eine erste Sensoreinheit (7) vorgesehen ist, die als bildgebende Sensoreinheit ausgebildet ist und die eine Vitalität der biologischen Mikroorganismen (6) erfasst, und
dass eine mit der Sensoreinheit (7) in Kommunikation stehende, ein Bildauswertemodul aufweisende, Auswerteinheit (9) vorgesehen ist, in der seitens der Sensoreinheit (7) generierte Sensorsignale unter Zugrundelegung wenigstens einer Auswertevorschrift auswertbar sind und mittels der bei Eintreten eines vorgebbaren Entscheidungskriteriums ein Signal erzeugbar ist.

3. Vorrichtung nach Anspruch 1 oder 2,
**dadurch gekennzeichnet, dass** wenigstens eine zweite Sensoreinheit (7') längs einer gegen äußere Einflüsse gesicherte Referenztrinkwasser führenden Leitung (10) vorgesehen ist, in der dem Referenztrinkwasser gleichfalls das Indikatormedium zugesetzt ist, das von der zweiten Sensoreinheit (7') optisch erfassbar ist,
dass die zweite Sensoreinheit (7') in Kommunikation mit der Auswerteinheit (9) steht und die Seitens der zweiten Sensoreinheit (7') generierten Sensorsignale gemeinsam mit den Sensorsignalen der ersten Sensoreinheit (7) unter Zugrundelegung wenigstens einer Auswertevorschrift auswertbar sind.

4. Vorrichtung nach einem der Ansprüche 1 bis 3,
**dadurch gekennzeichnet, dass** die erste Sensoreinheit (7) spektralempfindlich und/oder Polarisationsempfindlich ist.

5. Vorrichtung nach Anspruch 1
**dadurch gekennzeichnet, dass** wenigstens eine Lichtquelle vorgesehen ist, mittels der von der ersten Sensoreinheit (7) optisch erfassbare Durchströmungsvolumen beleuchtbar ist.

6. Vorrichtung nach einem der Ansprüche 1 bis 5
**dadurch gekennzeichnet, dass** mittels der ersten Sensoreinheit (7) die biologischen Mikroorganismen (6) direkt oder mittels einer Vergrößerungsoptik erfassbar sind.

7. Vorrichtung nach Anspruch 3,
**dadurch gekennzeichnet, dass** die zweite Sensoreinheit (7') baugleich zur ersten Sensoreinheit (7) ausgebildet ist.

8. Verfahren zur Online-Kontrolle von Trinkwasser auf Humanverträglichkeit innerhalb eines Trinkwasserversorgungsnetzes mit wenigstens einer Trinkwasserspeisestelle unter Verwendung einer Vorrichtung nach einem der Ansprüche 1, 3 bis 7,
**dadurch gekennzeichnet, dass** dem Trinkwasser innerhalb eines räumlich begrenzten Leitungsbereiches biologische Mikroorganismen (6) zugegeben werden, deren Vitalität mittels einer ersten bildgebenden Sensoreinheit (7) erfasst wird, deren Bildsensorsignale im Rahmen einer Bildauswertung unter Zugrundelegung einer Auswertevorschrift analysiert werden und dass bei Entsprechen der Bildsensorsignale eines vorgebbaren Entscheidungskriteriums ein Signal erzeugt wird.

9. Verfahren zur Online-Kontrolle von Trinkwasser auf Humanverträglichkeit innerhalb eines Trinkwasserversorgungsnetzes mit wenigstens einer Trinkwasserspeisestelle unter Verwendung einer Vorrichtung nach einem der Ansprüche 2 bis 7, **dadurch gekennzeichnet, dass** der dem Trinkwasser entnommenen Trinkwasserprobe biologische Mikroorganismen (6) zugegeben werden, deren Vitalität mittels einer ersten bildgebenden Sensoreinheit (7) erfasst wird, deren Bildsensorsignale im Rahmen einer Bildauswertung unter Zugrundelegung einer Auswertevorschrift analysiert werden und dass bei Entsprechen der Bildsensorsignale eines vorgebbaren Entscheidungskriteriums ein Signal erzeugt wird.

10. Verfahren nach Anspruch 8 oder 9,
**dadurch gekennzeichnet, dass** die biologischen Mikroorganismen (6) im Rahmen der Bildauswertung auf Veränderungen hinsichtlich ihrer Farbe, Beweglichkeit und/oder räumlichen Verteilung untersucht werden.

11. Verfahren nach Anspruch 8 oder 10,
**dadurch gekennzeichnet, dass** der räumlich begrenzte Leitungsbereich hinsichtlich einer durch die Mikroorganismen (6) bedingten Trübung optisch erfasst wird.

12. Verfahren nach Anspruch 9 oder 10, **dadurch gekennzeichnet, dass** die Trinkwasserprobe hinsichtlich einer durch die Mikroorganismen (6) bedingten Trübung optisch erfasst wird.

13. Verfahren nach einem der Ansprüche 8 bis 12 ,
**dadurch gekennzeichnet, dass** eine gegen äußere Einflüsse gesicherte Referenztrinkwasserprobe, in der gleichfalls die biologische Mikroorganismen (6) beigegeben werden, mittels einer zweiten optischen Sensoreinheit (7') untersucht wird, und
dass die von der der zweiten Sensoreinheit (7') generierten Sensorsignale gemeinsam mit den Sensorsignalen der ersten Sensoreinheit (7) unter Zugrundelegung wenigstens einer Auswertevorschrift ausgewertet werden.

14. Verfahren nach Anspruch 13,
**dadurch gekennzeichnet, dass** die Sensorsignale der ersten und zweiten Sensoreinheit (7, 7') im Wege des Differenzprinzips ausgewertet werden.

## Claims

1. A device for online checking of drinking water on human tolerance, which device can be provided on at least one drinking water line portion, through which drinking water can be passed, within a drinking water supply network having at least one drinking water feed point, to which a plurality of drinking water lines (1) are indirectly or directly connected and lead to local drinking water consumer points,
**characterised in that** the device comprises an indicator medium containing biological microorganisms, **in that** the device comprises a semi-permeable limiting means (4) suitable for use in the drinking water line portion, wherein, when inserted, the semi-permeable limiting means (4) limits a flow volume of the drinking water line portion at least on one side in the flow direction,
and wherein the limiting means (4) localises at least the indicator medium, which contains biological microorganisms (6) and can be introduced into the flow volume, since the limiting means (4) holds back the indicator medium within the flow volume against the flow direction,
**in that** a first sensor unit (7) is provided outside the flow medium in order to optically detect the indicator medium and is formed as an imaging sensor unit and detects a vitality of the biological microorganisms (6), and
**in that** an evaluation unit (9) in communication with the sensor unit (7) and having an image evaluation module is provided, in which evaluation unit sensor signals generated by the sensor unit (7) can be evaluated on the basis of at least one evaluation rule, and by means of which evaluation unit a signal can be generated in the event that a predefinable decision criterion occurs.

2. A device for online checking of drinking water on human tolerance, which device can be provided on at least one drinking water line portion, through which drinking water can be passed, within a drinking water supply network having at least one drinking water feed point, to which a plurality of drinking water lines (1) are indirectly or directly connected and lead to local drinking water consumer points,
**characterised in that** at least one automatically closable drain (11) is provided, by means of which a drinking water sample can be discharged in portions from the drinking water supply network,
**in that** at least one storable vessel (12) is provided, into which the drinking water sample can be introduced via the drain (11),
**in that** a feed unit containing an indicator medium containing biological microorganisms (6) is provided, by means of which feed unit the indicator medium containing the biological microorganism (6) can be introduced into the vessel (12) filled with the drinking water sample,
**in that** a first sensor unit (7) is provided outside the vessel (12) in order to optically detect the indicator medium and is formed as an imaging sensor unit and detects a vitality of the biological microorganisms (6), and
**in that** an evaluation unit (9) in communication with the sensor unit (7) and having am image evaluation module is provided, in which evaluation unit sensor signals generated by the sensor unit (7) can be evaluated on the basis of at least one evaluation rule, and by means of which evaluation unit a signal can be generated in the event that a predefinable decision criterion occurs.

3. The device according to Claim 1 or 2,
**characterised in that** at least one second sensor unit (7') is provided along a line (10) conveying reference drinking water and protected against external influences, in which line the indicator medium is also added to the reference drinking water and can be optically detected by the second sensor unit (7'),
**in that** the second sensor unit (7') is in communication with the evaluation unit (9), and the sensor signals generated by the second sensor unit (7') can be evaluated together with the sensor signals of the first sensor unit (7) on the basis of at least one evaluation rule.

4. The device according to one of Claims 1 to 3,
**characterised in that** the first sensor unit (7) has spectral sensitivity and/or polarisation sensitivity.

5. The device according to Claim 1,
**characterised in that** at least one light source is provided, by means of which the flow volume, which is optically detectable by the first sensor unit (7), can be illuminated.

6. The device according to one of Claims 1 to 5,
**characterised in that** the biological microorganisms (6) are detectable directly or by means of a magnification optics by means of the first sensor unit (7).

7. The device according to Claim 3,
**characterised in that** the second sensor unit (7') is structurally identical to the first sensor unit (7).

8. A method for online checking of drinking water on human tolerance within a drinking water supply network having at least one drinking water feed point with use of a device according to one of Claims 1, 3 to 7,
**characterised in that** biological microorganisms (6) are added to the drinking water within a spatially limited line area,
the vitality of said biological microorganisms is detected by means of a first imaging sensor unit (7), of which the image sensor signals are analysed within the scope of an image evaluation on the basis of an evaluation rule, and **in that** a signal is generated in the event that the image sensor signals correspond to a predefinable decision criterion.

9. A method for online checking of drinking water on human tolerance within a drinking water supply network having at least one drinking water feed point with use of a device according to one of Claims 2 to 7,
**characterised in that** biological microorganisms (6) are added to the drinking water samples taken from the drinking water, the vitality of said biological microorganisms is detected by means of a first imaging sensor unit (7), of which the image sensor signals are analysed within the scope of an image evaluation on the basis of an evaluation rule, and **in that** a signal is generated in the event that the image sensor signals correspond to a predefinable decision criterion.

10. The method according to Claim 8 or 9,
**characterised in that** the biological microorganisms (6) are examined within the scope of the image evaluation for changes in respect of their colour, mobility and/or spatial distribution.

11. The method according to Claim 8 or 10,
**characterised in that** the spatially limited line area is optically detected in respect of a turbidity caused by the microorganisms (6).

12. The method according to Claim 9 or 10,
**characterised in that** the drinking water sample is optically detected in respect of a turbidity caused by the microorganisms (6).

13. The method according to one of Claims 8 to 12,
**characterised in that** a reference drinking water sample protected against external influences and to which the biological microorganisms (6) are also added is examined by means of a second optical sensor unit (7'), and
**in that** the sensor signals generated by the second sensor unit (7') are evaluated together with the sensor signals of the first sensor unit (7) on the basis of at least one evaluation rule.

14. The method according to Claim 13,
**characterised in that** the sensor signals of the first and second sensor unit (7, 7') are evaluated by means of the differential principle.

## Revendications

1. Dispositif pour le contrôle en ligne d'eau potable quant à l'aptitude à la consommation humaine, lequel peut être prévu au niveau d'au moins un tronçon de conduite d'eau potable pouvant être traversé par l'eau potable à l'intérieur d'un réseau de distribution d'eau potable qui présente au moins un point d'alimentation en eau potable auquel sont reliées directement ou indirectement une pluralité de conduites d'eau potable (1) conduisant vers des points de consommation d'eau potable décentralisés,
**caractérisé en ce que** le dispositif comprend un milieu indicateur contenant des micro-organismes, **en ce que** le dispositif comprend un moyen de délimitation (4) semi-perméable adapté pour l'utilisation dans le tronçon de conduite d'eau potable, dans lequel, lors de l'utilisation, le moyen de délimitation semi-perméable (4) limite un volume de passage de flux du tronçon de conduite d'eau potable au moins d'un côté en direction du flux,
et dans lequel le moyen de délimitation (4) localise au moins le milieu indicateur, contenant des micro-organismes biologiques (6), pouvant être introduit dans le volume de passage de flux, **en ce que** le moyen de délimitation (4) retient le milieu indicateur à l'intérieur du volume de passage de flux à l'encontre de la direction du flux,
**en ce que**, pour la détection optique du milieu indicateur en-dehors du volume de passage de flux, une première unité de détection (7) est prévue, laquelle est réalisée en tant qu'unité de détection d'imagerie et laquelle détecte une vitalité des micro-organismes biologiques (6), et
**en ce que** l'on prévoit une unité d'évaluation (9) en communication avec l'unité de détection (7), présentant un module d'évaluation d'image, dans laquelle il est possible d'évaluer des signaux de détection générés du côté de l'unité de détection (7) en prenant pour base au moins une règle d'évaluation, et au moyen de laquelle un signal peut être généré en cas d'occurrence d'un critère de décision prescriptible.

2. Dispositif pour le contrôle en ligne d'eau potable quant à l'aptitude à la consommation humaine, lequel peut être prévu au niveau d'au moins un tronçon de conduite d'eau potable pouvant être traversé par l'eau potable à l'intérieur d'un réseau de distribution d'eau potable qui présente au moins un point d'alimentation en eau potable auquel sont reliées directement ou indirectement une pluralité de conduites d'eau potable (1) conduisant vers des points de consommation d'eau potable décentralisés,
**caractérisé en ce que** le dispositif comprend au moins une dérivation des eaux (11) pouvant être fermée automatiquement à l'aide de laquelle un échantillon d'eau potable est prélevé par portions du réseau de distribution d'eau potable,
**en ce que** l'on prévoit au moins un récipient (12) pouvant être alimenté, dans lequel l'échantillon d'eau potable peut être introduit via la dérivation des eaux (11),
**en ce que** l'on prévoit une unité d'addition contenant un milieu indicateur contenant des micro-organismes biologiques (6) à l'aide de laquelle le milieu indicateur contenant les micro-organismes biologiques (6) peut être introduit dans le récipient (12) rempli avec l'échantillon d'eau potable,
**en ce que**, pour la détection optique du milieu indicateur en-dehors du récipient (12), une première unité de détection (7) est prévue, laquelle est réalisée en tant qu'unité de détection d'imagerie et laquelle détecte une vitalité des micro-organismes biologiques (6), et
**en ce que** l'on prévoit une unité d'évaluation (9) en communication avec l'unité de détection (7), présentant un module d'évaluation d'image, dans laquelle il est possible d'évaluer des signaux de détection générés du côté de l'unité de détection (7) en prenant pour base au moins une règle d'évaluation et au moyen de laquelle un signal peut être généré en cas d'occurrence d'un critère de décision prescriptible.

3. Dispositif selon la revendication 1 ou 2,
**caractérisé en ce que** l'on prévoit au moins une deuxième unité de détection (7') le long d'une conduite (10) conduisant une eau potable de référence protégée contre les influences extérieures dans laquelle on ajoute également le milieu indicateur à l'eau potable de référence, lequel peut être détecté optiquement par la deuxième unité de détection (7'),
**en ce que** la deuxième unité de détection (7') est en communication avec l'unité d'évaluation (9) et **en ce que** les signaux de détection générés du côté de la deuxième unité de détection (7') peuvent être évalués ensemble avec les signaux de détection de la première unité de détection (7) en prenant pour base au moins une règle d'évaluation.

4. Dispositif selon l'une des revendications 1 à 3,
**caractérisé en ce que** la première unité de détection (7) est à sensibilité spectrale et/ou à sensibilité de polarisation.

5. Dispositif selon la revendication 1,
**caractérisé en ce que** l'on prévoit au moins une source lumineuse à l'aide de laquelle il est possible d'éclairer des volumes de passage de flux détectables optiquement par la première unité de détection (7).

6. Dispositif selon l'une des revendications 1 à 5,
**caractérisé en ce que**, grâce au premier dispositif de détection (7), les micro-organismes biologiques (6) peuvent être détectés directement ou au moyen d'une optique de grossissement.

7. Dispositif selon la revendication 3,
**caractérisé en ce que** la deuxième unité de détection (7') est réalisée avec une configuration identique à la première unité de détection (7).

8. Procédé pour le contrôle en ligne d'eau potable quant à l'aptitude à la consommation humaine au sein d'un réseau de distribution d'eau potable avec au moins un point d'alimentation en eau potable en utilisant un dispositif selon l'une des revendications 1, 3 à 7,
**caractérisé en ce que** l'on ajoute à l'eau potable, à l'intérieur d'une zone de conduite délimitée spatialement, des micro-organismes biologiques (6) dont la vitalité est mesurée au moyen d'une première unité de détection d'imagerie (7) dont les signaux de détection d'image sont analysés dans le cadre d'une évaluation d'image en prenant pour base une règle d'évaluation, et **en ce qu'**en cas de conformité des signaux de détection d'image d'un critère de décision prescriptible, un signal est généré.

9. Procédé pour le contrôle en ligne d'eau potable quant à l'aptitude à la consommation humaine au sein d'un réseau de distribution d'eau potable avec au moins un point d'alimentation en eau potable en utilisant un dispositif selon l'une des revendications 2 à 7,
**caractérisé en ce que** des micro-organismes biologiques (6) sont ajoutés à l'échantillon d'eau potable prélevé de l'eau potable dont la vitalité est détectée au moyen d'une première unité de détection d'imagerie (7) dont les signaux de détection d'image sont analysés dans le cadre d'une évaluation d'image en prenant pour base une règle d'évaluation, et **en ce qu'**en cas de conformité des signaux de détection d'image d'un critère de décision prescriptible, un signal est généré.

10. Procédé selon la revendication 8 ou 9,
**caractérisé en ce que** les micro-organismes biologiques (6) sont analysés dans le cadre de l'évaluation d'image quant à des modifications concernant leur couleur, mobilité et/ou répartition spatiale.

11. Procédé selon la revendication 8 ou 10,
**caractérisé en ce que** la zone de conduite délimitée spatialement est détectée optiquement en ce qui concerne une turbidité causée par les micro-organismes (6).

12. Procédé selon la revendication 9 ou 10,
**caractérisé en ce que** l'échantillon d'eau potable est détecté optiquement en ce qui concerne une turbidité causée par les micro-organismes (6).

13. Procédé selon l'une des revendications 8 à 12,
**caractérisé en ce qu'**un échantillon d'eau potable de référence protégé contre des influences extérieures, dans lequel on ajoute également les micro-organismes biologiques (6), est analysé au moyen d'une deuxième unité de détection optique (7'), et
**en ce que** les signaux de détection générés par la deuxième unité de détection (7') sont évalués ensemble avec les signaux de détection de la première unité de détection (7) en prenant pour base au moins une règle d'évaluation.

14. Procédé selon la revendication 13,
**caractérisé en ce que** les signaux de détection des première et deuxième unités de détection (7, 7') sont évalués selon le principe de différence.
